# EUROPÄISCHE PATENTSCHRIFT

(11) **EP 1 917 051 B1**
(45) Veröffentlichungstag und Bekanntmachung des Hinweises auf die Patenterteilung: **23.06.2010**
(21) Anmeldenummer: 06775783.1
(22) Anmeldetag: 02.08.2006
(51) Int. Cl.: A61L 27/56, A61F 2/28

(54) **SPONGIÖS-METALLISCHES IMPLANTAT UND VERFAHREN ZU SEINER HERSTELLUNG**
SPONGY METALLIC IMPLANT AND METHOD FOR THE PRODUCTION THEREOF
IMPLANT METALLIQUE-SPONGIEUX ET SON PROCEDE DE PRODUCTION

(30) Priorität: 06.08.2005 DE 102005037141
(43) Veröffentlichungstag der Anmeldung: 07.05.2008
(73) Patentinhaber: m.pore GmbH, 01277 Dresden (DE)
(72) Erfinder: GIRLICH, Dieter, 01309 Dresden (DE); DITTEL, Karl-Klaus, 70569 Stuttgart (DE)
(74) Vertreter: Kaufmann, Sigfrid
(86) Internationale Anmeldenummer: PCT/DE2006/001342
(87) Internationale Veröffentlichungsnummer: WO 2007/016902

(56) Entgegenhaltungen:
- EP-A1- 0 560 279
- WO-A-02/17820
- WO-A-99/16478
- WO-A2-00/64504
- DE-A1- 19 939 155
- US-A1- 2005 129 949

## Beschreibung

Die Erfindung betrifft den modularen Einsatz von offenporigen Metallschwämmen als alloplastischer Knochenersatz in der Unfallchirurgie und Orthopädie, deren Anwendung sich besonders zur dauerhaften Auffüllung posttraumatischer und osteoporose- bzw. tumorbedingter knöcherner Defekte älterer Patienten anbietet.

Mehr als 100.000 Knochentransplantationen pro Jahr in Deutschland beinhalten erhebliche volkswirtschaftliche Bedeutung. Die Auffüllung posttraumatischer oder tumorbedingter Knochendefekte stellt damit nicht zuletzt aufgrund der Altersstruktur der Bevölkerung ein zunehmendes klinisches Problem dar. Die Nachteile der autogenen Transplantation liegen in erforderlichen Zweiteingriffen und in der limitierten Verfügbarkeit des autogenen Materials. Andererseits bieten sich erwiesenermaßen metallische Substitute, besonders solche aus Cobalt-Chrom-Legierungen und Titan bzw. Titan-Legierungen, für vielfältige klinische Anwendungen an; mit den derzeitig verfügbaren Implantaten ist allerdings das besonders ältere Patienten physisch und psychisch stark belastende Problem eines Zweiteingriffes nur bedingt oder gar nicht gelöst.

Die Osteoporose bedeutet für das betreffende Patientengut eine Reduktion der Knochenmasse mit Rarefizierung der Bälkchenstruktur, wobei im Alter von 65 Jahren eine Reduktion der Knochendichte durch den Alterungsprozeß um 30% üblich ist. Es ist deshalb nicht überraschend, dass pathologische osteoporotisch bedingte Frakturen mit zunehmenden Alter exponentiell ansteigen und die Versorgung dieser Frakturen vielfach deshalb so schwierig ist, weil verbliebene Defekte nach Reposition nur in unbefriedigender Weise zu rekonstruieren bzw. aufzufüllen sind.

Frakturen infolge eines inadäquaten Traumas sind meistens Folge einer zuvor unerkannten und unbehandelten Osteoporose und betreffen etwa die Hälfte aller Frauen sowie ein Drittel aller Männer über 50 Jahre. Von zentraler Bedeutung ist dabei, dass Personen mit einer Osteoporose, die bereits eine Fraktur erlitten haben, einem relevant höherem Risiko für weitere Frakturen ausgesetzt sind und damit zu dem Problemkreis der zu versorgenden Patienten zu zählen sind.

Osteoporotische Frakturen können überall im Bereich des Stammskelettes und peripher auftreten, sind jedoch hüftgelenksnah, im Bereich der Wirbelsäule, des Handgelenkes und des proximalen Humerus am häufigsten zu finden.

Über individuelle und gesundheitliche Folgen hinaus, bedeuten osteoporosebedingte Frakturen direkt und in Form ihrer Folgekosten auch eine enorme sozio-ökonomische Belastung für die Gesellschaft. Ökonomische Schätzungen gehen davon aus, dass in der Bundesrepublik Deutschland mindestens 3 Mrd. EUR für die Behandlung der Osteoporose ausgegeben werden, wobei der Hauptteil auf die Versorgung nach eingetretener pathologischer Fraktur entfällt.

Bei den entwickelten Implantaten stand von Anfang an als Hauptproblem deren knöchernes An- bzw. Einwachsen. Im Gegensatz zu Implantaten mit aufgerauten, gestrahlten Oberflächen, die nur ein Knochenanwachsen ermöglichen, und Implantaten mit mikroporösen Oberflächen, meist erzeugt durch Plasmaspray, die ein begrenztes knöchernes Einwachsen erlauben, zielen die seit den 80er Jahren vorgestellten Implantate mit metall-spongiöser Oberfläche auf die vollständige knöcherne Integration im Sinne eines knöchernen Durchwachsens. Derartige Implantate als Ersatz für spongiöse Knochen sind namentlich aus DE 31 06 917 C2, DE 32 24 265 C2, DE 39 17 033 C1 und DE 41 06 971 C1 bekannt.

Zunächst wurde eine schwammartige, zweidimensionale Oberflächenstruktur vorgeschlagen, die eine Porenweite von 0,5 bis 1,5 mm aufwies, jedoch keine räumliche Verbindung in der metallischen Tiefe aufwies; infolge dessen konnte nur ein Heran- und Hineinwachsen des Knochens, jedoch kein knöchernes Hindurchwachsen erzielt werden. Mit DE 32 24 265 C2 wurde ein dreidimensionales Implantat vorgestellt, dessen metall-spongiöse Oberflächenstruktur eine Einheit mit einem massiven Metallkern bildet. Das so strukturierte Implantat hat eine Oberflächenporosität von ca. 60%; die Porengröße liegt zwischen 0,3 und 2,5 mm, die Höhe der Struktur beträgt ca. 3 mm. Die damit bereits 1981 vorgenommenen Tierversuche mit metallspongiösen Implantatwürfeln bestätigen die Annahme einer knöchernen Fusion mit dem Implantat und rechtfertigen 1982 die Anwendung auch am Menschen.

Um den mechanischen Beanspruchungen besser gerecht zu werden, wurde in DE 39 17 033 C1 ein Verfahren vorgeschlagen, das die Herstellung einer Struktur mit 0,9 bis 4,3 mm Porengröße und einer erhöhten Porosität von ca. 70% erlaubt; allerdings konnten verfahrensimmanent die technischen Parameter wie Höhe und Stegdicke der Struktur nicht beeinflußt werden, sodass, zumal die Parameter in der Praxis nicht reproduzierbar waren (Firmenschrift "ESKA Aktuell" E-10/05), letztlich diese Lösung praktisch nicht befriedigen konnte. Schließlich bietet DE 41 06 971 C1 ein Verfahren zur Herstellung eines Implantates mit einer seine Oberfläche zumindest teilweise bedenkende metallischen offenzelligen Struktur an, die sich durch symmetrisch angeordnete Stege auszeichnet. Die damit ausgerüsteten Hüft- und Knieprothesen ermöglichen den Knocheneinwuchs durch diese Trabekel dadurch, daß sie den Implantatschild gleichsam wie in einer Hängematte befestigen.

Der Stand der Technik ist des Weiteren besonders durch die Schriften DE 41 33 877 C1, DE 42 08 247 C1, DE 42 14 357 C2, DE 43 36 819 C1, DE 195 43 530 C1, DE 196 14 949 A1 und DE 199 07 489 C1, die Herstellungsverfahren, spezielle Implantate bzw. Hohlendoprothesen beschreiben, charakterisiert.

Für die vorliegende Erfindung steht die Aufgabe, ein formsteifes, biokompatibles und zugleich kostengünstig herstellbares Implantat mit spongiöser Struktur zu entwickeln, um Defekte in osteoporotischen Knochen oder Tumordefekte permeabel auszufüllen. Hierbei soll das Implantat so modifiziert sein, dass der Operateur intraoperativ für die jeweiligen Knochenverhältnisse auf eine spezielle Lösungsmöglichkeit zurückgreifen kann.

Diese Aufgabe wird durch die Merkmale des Anspruches 1 gelöst. Zweckmäßige Ausgestaltungen ergeben sich aus den Merkmalen der Ansprüche 2 bis 8.

Nach Maßgabe der Erfindung ist vorgesehen, dass das Implantat ausschließlich aus einer oder mehreren offenporigen Metallstrukturen besteht, die in Form eines standardisierten Baukastensystems modular aufgebaut sind. Hierfür ist der Metallschwamm keil-, quader-, zylinder-, ring-, scheiben- und/oder rautenförmig ausgeformt. Die Module, die sich verschiedenartig kombinieren lassen, liegen jeweils in unterschiedlichen Größen vor, sodass die damit gebildeten Implantate befundgerecht zur Ausfütterung der o. g. Defekte eingepasst werden können. Die Möglichkeit, unterschiedliche, vorgefertigte Formvarianten situationsgerecht zu konnektieren, ist ein wesentlicher Vorteil der Erfindung. Es ist aber auch ohne weiteres möglich, diese Module bzw. das komplette Implantat nach einer CT-Auswertung (custom made) individuell auszuformen. Dem Wesen der Erfindung folgend ist vorgesehen, die Module und/oder Teile von ihnen kraft- und/oder formschlüssig miteinander zu kombinieren. Dabei können die Module vorteilhafterweise durch Klemm- bzw. Klettverschlüsse oder durch Schrauben, die gleichfalls aus offenporigen, gleichartigen Metallschwamm bestehen, konnektiert sein.

Der das Implantat bildende Metallschwamm besteht bevorzugt aus Titan oder einer Titanlegierung und zeichnet sich durch eine sehr hohe Porosität aus. Es hat sich überraschend gezeigt, daß mit Titan, welches sowohl biokompatibel, nicht tumorinduktiv als auch osteokonduktiv ist, sehr wohl formsteife Implantate mit einer Porosität von über 90% herstellbar sind. Es kann begründet angenommen werden, dass auch mit den grundsätzlich ähnlich gut geeigneten Cobalt-Legierungen Implantate, die ausschließlich aus einer Schwammstruktur bestehen, erzeugt werden können. Obgleich bei der Verwendung eines Titan-Schwamm-Implantates eigentlich keine Biofunktionalisierung notwendig ist, kann es im Einzelfall vorteilhaft sein, die Struktur mit Hydroxylapatit oder Tantal zu beschichten, wobei eine Dicke von 5 µm, maximal 8 µm ausreichend ist.

Der zentrale Vorteil der vorliegenden Erfindung ist, dass ein vollständig permeables Implantat vorliegt, welches im spongiösen Knochen in Abhängigkeit von der erforderlichen Stabilität dem individuell erforderlichen Integrationsumfang gewährleisten kann. Aufgrund der vollständigen Offenporigkeit und der Steifigkeit des Implantates besteht im belasteten Knochen die Möglichkeit der durchgreifenden knöchernen Integration. Bestimmend für den praktischen Einsatz des Implantates ist, dass tatsächlich - neben einer sehr hohen Porosität - die erforderliche Stabilität in hohem Maße reproduzierbar und zugleich kostengünstig herstellbar ist; unumgänglich ist dabei, dass die Stege der Struktur vergleichsweise dick sind.

Unter diesen Aspekten ist vorgesehen, dass das Implantat aus einer retikulierten Struktur, bevorzugt einer retikulierten Schwammstruktur, wobei die durch solche Strukturen gebildeten Gießkanäle vor dem Einfüllen des Metalls mittels organischer Materialien stabilisiert werden, hergestellt wird und die Stege der retikulierten Strukturen durch ein- oder mehrmaliges Beschichten verdickt werden. Das zur Beschichtung der Stege dienende organische Material kann flüssiges Wachs, Wachspulver, Polystyrol-Pulver oder Polyamid-Pulver sein, wobei das Polystyrol/Polyamid einen geringen, bevorzugt 5%igen Anteil Kleber impliziert. Bei mehrfacher Beschichtung der Stege werden die einzelnen Beschichtungen bei unterschiedlichen Temperaturen im Bereich von 40 bis 80°C aufgebracht.

Mit der Erfindung liegt erstmals ein Implantat vor, das eine durchgreifende knöcherne Integration ermöglicht. Durch die knöcherne Langzeitintegration des Implantates kann - neben den o. g. dargestellten Vorzügen - das Entstehen verbleibender sekundärer Fehlstellungen in osteoporotischen Knochen wirksam vermieden werden. Konkrete bestimmungsgemäße Verwendungsmöglichkeiten des vorgeschlagenen Implantates sind beispielsweise:
- Proximale pathologische Tibiafraktur bei schwerer Osteoporose (Belastungsunfähigkeit)
- Distale pathologische Femurfraktur nach Marknagelung
   - Durchgeführte Therapie: Zweimalige Defektauffüllung mit offenporigem Titan-Metallschwamm und additiver Plattenosteosynthese
- Fibuladefektsituation bei metastasierendem Mammacarcinom mit Instabilität (massiv eingeschränkte Geh- und Belastungsfähigkeit)
   - Durchgeführte Therapie: Metastasenresektion und Defektauffüllung mit offenporigem Titan-Metallschwamm mit additiver Osteosynthese
- Acetabuläre Defektsituation nach zementierter Hüftendoprothese (Gehunfähigkeit)
   - Durchgeführte Therapie: Endoprothesenwechselsituation. Defektauffüllung mit offenporigem Titan-Metallschwamm und Hüftpfannenrekonstruktion durch Metallacetabulum

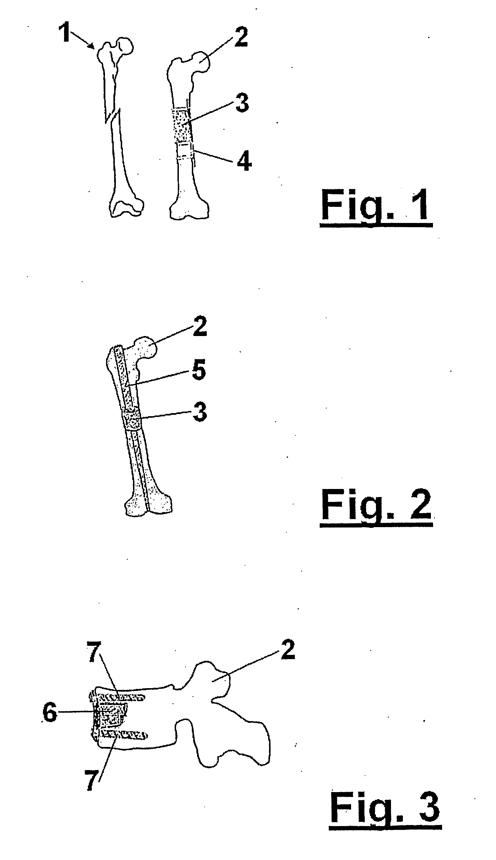

Die Anwendung der Erfindung wird des Weiteren anhand von schematischen Darstellungen näher erläutert. Hierfür zeigen
- Fig. 1 das Prinzip der Reparatur eines Knochendefektes 1 mit einem Implantat 3, bestehend ausschließlich aus einem Bausatz von Modulen aus der Titanlegierung Ti6AI4V und einer Schiene 4, die den Knochen 2 und das Implantat 3 lagestabilisiert.
- Fig. 2 ein Beispiel, bei dem der Knochen 2 durch einen Nagel 5 stabilisiert und ein Teil des Knochens komplett durch die Schwammstruktur 3 ersetzt wird.
- Fig. 3 gibt ein Beispiel, bei dem der Knochen 2 durch Implantatmodule 6 aufgefüllt wird, die ihrerseits mittels Schrauben 7, die gleichfalls aus Titanschwamm bestehen, fixiert werden.

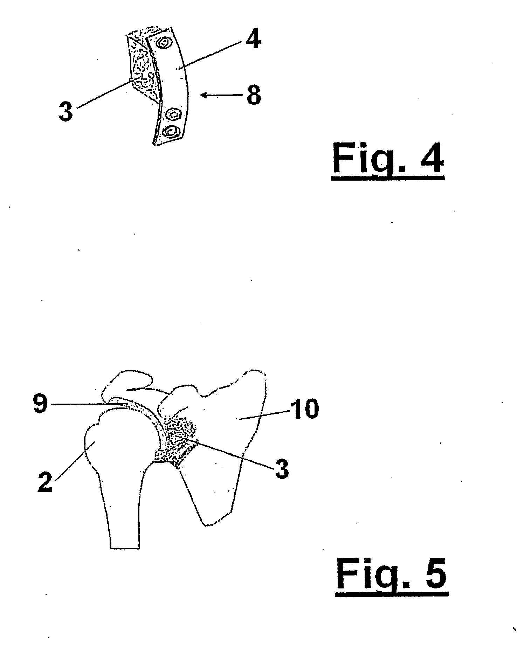

- Fig. 4 ein stoffschlüssiges Implantat 8 bestehend aus dem Titanschwamm 3 und der Titanschiene 4, hergestellt nach dem beschriebenen Gussverfahren.
- Fig. 5 eine Pfanne 9 für ein künstliches Hüftgelenk 10, das durch ein Titanschwamm-Implantat 3 stabilisiert wird.

### Liste der verwendeten Bezugszeichen

- 1: Knochendefekt
- 2: Knochen
- 3: Implantat aus Ti6Al4V-Schwammstruktur
- 4: Schiene
- 5: Nagel
- 6: Implantatmodule
- 7: Schraube aus Titanschwamm
- 8: stoffschlüssiges Implantat Metallschwamm/Schiene
- 9: Pfanne
- 10: künstliches Hüftgelenk

## Patentansprüche

1. Spongiös-metallisches Implantat (3, 6) als alloplastischer Knochenersatz aus offenporigem, hochfestem Metallschwamm, beispielsweise aus Titan oder einer Titanlegierung, **dadurch gekennzeichnet, dass** der Schwamm eine Porosität von 75 % bis 94 % aufweist, graduiert ist, wobei die Porengröße stetig von innen nach außen ansteigt, und der Schwamm derart konfektioniert ist, dass er aus mehreren konnektierten Modulen eines Baukastensystems oder aus einem Modul, dass auf der Basis einer CT-Auswertung individuell ausgeformt ist, besteht.

2. Implantat nach Anspruch 1 und einem der vorstehenden Ansprüche, **dadurch gekennzeichnet, dass** die Module und/oder Teile von ihnen kraft- und/oder formschlüssig miteinander kombinierbar sind.

3. Implantat nach Anspruch 2, **dadurch gekennzeichnet, dass** die Module durch einen Klemm- oder Klettverschluss verbunden sind.

4. Implantat nach Anspruch 3, **dadurch gekennzeichnet, dass** die Module durch Schrauben, die eine gleichartige offenporige Metallstruktur aufweisen, verbunden sind.

5. Implantat nach Anspruch 1 und einem der vorstehenden Ansprüche, **dadurch gekennzeichnet, dass** die Module des standardisierten Baukastensystems keil-, quader-, zylinder-, ring-, scheiben-, kugel- und/oder rautenförmig ausgeformt sind und jeweils in unterschiedlicher Größe vorliegen.

6. Implantat nach Anspruch 1 bis 5, **dadurch gekennzeichnet, dass** die Metallschwammstruktur mit Hydroxylapatit beschichtet ist.

7. Implantat nach Anspruch 1 bis 5, **dadurch gekennzeichnet, dass** die Metallschwammstruktur mit Tantal beschichtet ist.

8. Implantat nach Anspruch 1 bis 5, **dadurch gekennzeichnet, dass** die Metallschwammstruktur mit Calcium-Phosphat beschichtet ist.

## Claims

1. A spongy metallic implant (3, 6) as alloplastic bone replacement comprising open-pore, high-strength metal sponge, for example of titanium or a titanium alloy, **characterised in that** the sponge displays a porosity of 75% to 94%, is graduated, wherein the pore size increases constantly from the inside to the outside, and the sponge is made up in such manner that it comprises several connected modules of a modular system or a module formed individually on the basis of a CT evaluation.

2. An implant according to Claim 1, **characterised in that** the modules and/or parts thereof can be combined with each other non-positively and/or positively.

3. An implant according to Claim 2, **characterised in that** the modules are connected by way of a clamping or interlocking fastener.

4. An implant according to Claim 3, **characterised in that** the modules are connected by way of screws which display a like open-pore metal structure.

5. An implant according to Claim 1 and one of the preceding claims, **characterised in that** the modules of the standardised modular system are formed in wedge, block, cylinder, ring, disc, ball and/or rhombus form and are each present in different sizes.

6. An implant according to Claims 1 to 5, **characterised in that** the metal sponge structure is coated with hydroxylapatite.

7. An implant according to Claims 1 to 5, **characterised in that** the metal sponge structure is coated with tantalum.

8. An implant according to Claims 1 to 5, **characterised in that** the metal sponge structure is coated with calcium phosphate.

## Revendications

1. Implant métallique spongieux (3, 6) en tant que substitut d'os alloplastique en éponge métallique haute résistance à pores ouverts, par exemple en titane ou en alliage de titane, **caractérisé en ce que** la porosité de l'éponge est échelonnée de 75 % à 94 %, la taille des pores augmentant de manière continue de l'intérieur à l'extérieur, et que l'éponge est confectionnée de manière à être constituée d'une jonction de plusieurs modules d'un système modulaire ou d'un seul module de forme personnalisée, défini sur la base de l'évaluation d'un scanner.

2. Implant selon la revendication 1, **caractérisé en ce que** les modules et/ou des parties des modules peuvent être combinés entre eux de manière à former une unité du point de vue de la forme et/ou des propriétés statiques.

3. Implant selon la revendication 2, **caractérisé en ce que** les modules sont joints par serrage ou par une fermeture de type Velcro.

4. Implant selon la revendication 3, **caractérisé en ce que** les modules sont joints par des vis présentant une structure métallique à pores ouverts de même type.

5. Implant selon la revendication 1 et l'une des revendications précédentes, **caractérisé en ce que** les modules du système modulaire standardisé sont réalisés en forme de coin, de parallélépipède, de cylindre, d'anneau, de disque, de sphère et/ou de losange et qu'ils sont disponibles dans différentes tailles.

6. Implant selon les revendications 1 à 5, **caractérisé en ce que** la structure métallique spongieuse est recouverte d'hydroxylapatite.

7. Implant selon les revendications 1 à 5, **caractérisé en ce que** la structure métallique spongieuse est recouverte de tantale.

8. Implant selon les revendications 1 à 5, **caractérisé en ce que** la structure métallique spongieuse est recouverte de phosphate de calcium.
